# FASCICULE DE BREVET EUROPEEN

(11) **EP 0 365 440 B1**
(45) Date de publication et mention de la délivrance du brevet: **07.07.1993**
(21) Numéro de dépôt: 89420382.7
(22) Date de dépôt: 09.10.1989
(51) Int. Cl.: C07C 39/04, C07C 37/08, C07C 7/156, C07C 45/53, C07C 45/85

(54) **Procédé de destruction des péroxydes d'acétone**
Verfahren zur Vernichtung von Peroxiden in Aceton
Method of destroying peroxides in acetone

(30) Priorité: 14.10.1988 FR 8814360
(43) Date de publication de la demande: 25.04.1990
(73) Titulaire: RHONE-POULENC CHIMIE, 92408 Courbevoie Cédex (FR)
(72) Inventeur: Costantini, Michel, F-69003 Lyon (FR)
(74) Mandataire: Dutruc-Rosset, Marie-Claude

(56) Documents cités:
- EP-A- 0 018 803
- DE-A- 637 653
- DE-A- 2 165 027
- WORLD PATENT INDEX DATABASE, no. d'accession 80-50775C, semaine 29, 1980, sections E14,J01, Derwent Publications Ltd., London, GB; & JP-A-55 73 796
- WORLD PATENT INDEX (LATEST) DATABASE, no. d'accession 83-788014, semaine 41, 1983, sections A41,D15,E19, Derwent Publications Ltd., London, GB; SU-A-98 1247
- WORLD PATENT INDEX DATABASE, no. d'accession 80-50774C, semaine 29, 1980, sections E14,J01, Derwent Publications, London, GB; & JP-A-55 73 795

## Description

La présente invention concerne un procédé de destruction des peroxydes d'acétone.

Dans certains procédés de préparation de phénols et de diphénols notamment, où se trouvent en contact de l'acétone et un agent d'oxydation tel que le peroxyde d'hydrogène, on constate la formation de peroxydes d'acétone.

Ainsi dans la synthèse industrielle du phénol à partir du cumène, il est généré en fin de réaction du phénol, de l'acétone et des sous-produits tels que le phényl-2 propanol-2. Il est intéressant d'essayer d'améliorer la productivité du procédé en phénol et il a été notamment proposé d'oxyder le phényl-2 propanol-2 en hydroperoxyde de cumène, qui lui-même se scinde en phénol + acétone. Cette oxydation peut se faire notamment par addition de peroxyde d'hydrogène, comme décrit dans le brevet FR 1 077 475, ce qui conduit à la formation de peroxydes d'acétone.

De même, dans le procédé de préparation de l'hydroquinone à partir du diisopropylbenzène décrit dans EP-A-21 848, on retrouve le souci d'améliorer la productivité et la solution consistant en l'addition de peroxyde d'hydrogène, afin d'oxyder en bis(hydroperoxyde) de diisopropylbenzène certains sous-produits. Cela conduit comme précédemment à la formation dans le milieu réactionnel de peroxydes d'acétone.

Un autre exemple de procédé dans lequel se forment des peroxydes d'acétone est le procédé d'hydroxylation du phénol, de phénols substitués ou d'éthers du phénol, par le peroxyde d'hydrogène, en milieu acétonique et en présence de zéolites synthétiques, décrit dans le brevet français publié sous le numéro 2 523 575. Ce procédé permet de préparer des diphénols, des diphénols substitués ou des phénols substitués. Or, lors de la distillation de l'acétone, formée lors de la réaction ou utilisée comme solvant, et de la distillation du phénol ou des diphénols formés, les peroxydes d'acétone produits par réaction entre l'agent oxydant, tel que le peroxyde d'hydrogène, et l'acétone sont soit en partie concentrés dans le résidu de distillation, soit entrainés en partie et peuvent cristalliser dans différentes parties de l'appareillage.

Il y a donc potentiellement un danger d'explosion.

Par ailleurs, on a décrit dans JP-A-55073795 et JP-A-5507796 un procédé pour récupérer un solvant utilisé dans une réaction d'oxydation qui contient des peroxydes d'acétone. Ledit procédé consiste à traiter le solvant soit à l'aide d'un halogénure ou d'un hydroxyde de Co, Mn, Ni, Cr, Cu, V ou soit à l'aide d'un acide fort ayant un pka inférieur à 1,0.

L'objet de la présente invention est de fournir un procédé de destruction des peroxydes d'acétone dans un mélange réactionnel, avant une éventuelle distillation d'un ou plusieurs des constituants de ce mélange.

Plus précisément l'invention consiste en un procédé de destruction des peroxydes d'acétone présents dans un mélange réactionnel issu, notamment de la synthèse du phénol, de diphénols ou de dérivés du phénol, caractérisé en ce que :
- l'on amène le pH dudit mélange réactionnel à une valeur de 4 à 8,
- l'on ajoute audit mélange un composé du cuivre
- et l'on maintient ledit mélange à une température de 50°C à 150°C.

L'ordre de réalisation des phases du procédé peut être très variable :
- ajout du composé du cuivre, puis ajustement du pH et chauffage ;
- ajustement du pH, chauffage, puis ajout du composé du cuivre ;
- ajustement du pH, ajout du composé du cuivre, puis chauffage.

Les peroxydes d'acétone sont essentiellement constitués de 2 composés :
- le dimère formé à partir de 2 molécules d'acétone,
- le trimère formé à partir de 3 molécules d'acétone.

Dans un milieu constitué essentiellement de phénol ou de diphénols, d'acétone, d'un peu d'eau et de divers produits secondaires en relativement faibles quantités, la mesure d'un pH n'est guère possible et n'est pas significative.

Aussi dans le présent texte, nous utiliserons le terme pH en définissant les conditions de sa mesure.

Le pH du mélange réactionnel se définira comme le pH que l'on mesurera sur un mélange artificiel constitué par 2 cm3 dudit mélange réactionnel et de 10 cm3 d'eau;
Toutes les indications de pH données dans le présent texte correspondront donc à des valeurs déterminées comme indiqué ci-avant, sans que cela soit précisé chaque fois.

Les meilleurs résultats sont obtenus lorsque l'on opère à un pH compris entre 5 et 7.

Les différents procédés conduisant à la formation de peroxydes d'acétone sont mis en oeuvre en présence d'un catalyseur acide.

Pour les procédés mettant en oeuvre une catalyse acide homogène, l'ajustement du pH du mélange réactionnel à traiter aux valeurs indiquées précédemment se fera donc généralement par neutralisation de l'acidité dudit mélange réactionnel.

Cette neutralisation peut se faire selon tout moyen connu utilisé habituellement.

En pratique un moyen très simple est d'utiliser par exemple une solution aqueuse de soude.

Pour les procédés mettant en oeuvre une catalyse acide hétérogène (silicalite de titane par exemple) l'ajustement du pH se fera à l'aide d'une solution acide.

Les composés du cuivre, que l'on peut utiliser dans le procédé de l'invention, sont les composés cuivreux ou cuivriques, qui sont au moins partiellement solubles dans le mélange réactionnel à traiter.

Compte-tenu du caractère oxydant du milieu, le cuivre sera essentiellement sous forme cuivrique, quel que soit le degré d'oxydation sous lequel il a été introduit. C'est pourquoi il sera question dans le présent texte de composé cuivrique.

Parmi les composés du cuivre au moins partiellement solubles dans le mélange à traiter, on peut citer notamment les carboxylates cuivriques tels que par exemple l'acétate cuivrique, le propionate cuivrique, l'hexanoate cuivrique, l'octanoate cuivrique, le benzoate cuivrique ; les halogénures cuivriques tels que le chlorure cuivrique ; dans certains cas, la présence d'eau dans le milieu réactionnel permettra d'utiliser d'autres sels de cuivre, tels que le sulfate de cuivre ou le nitrate de cuivre.

L'acétate de cuivre convient tout particulièrement bien.

Si, comme c'est le cas le plus souvent, l'ajustement du pH du mélange réactionnel à traiter est réalisé avant l'addition du composé cuivrique, on peut, avant d'introduire ce dernier, séparer par filtration par exemple le sel formé lors de la neutralisation de l'acidité du mélange.

La quantité de composé cuivrique qui est utilisé dans le présent procédé peut varier dans de larges limites.

Habituellement on met en oeuvre de 0,05 à 5 moles de composé cuivrique pour 1 mole de peroxyde d'acétone.

De préférence le rapport molaire composé cuivrique/peroxydes d'acétone sera compris entre 0,1 et 2.

La température à laquelle est réalisée la destruction des peroxydes d'acétone est de préférence comprise entre 60°C et 130°C.

Généralement, pour éviter la distillation de composés les plus volatils du mélange réactionnel à traiter, on opérera en réacteur fermé, donc sous la pression autogène des différents réactifs.

De la température dépend la durée du traitement. Celle-ci peut varier de quelques minutes à une dizaine d'heures. Ces durées ne sont pas en elles-mêmes critiques, car elles dépendent de la température et de la teneur en peroxyde d'acétone.

Les 2 peroxydes d'acétone peuvent d'ailleurs avoir des comportements différents.

Le dimère est détruit par l'action des composés cuivriques, même à des températures relativement basses à condition d'opérer a un pH de 4 à 8. La destruction du trimère nécessite, soit la mise en oeuvre des conditions indiquées précédemment de température, d'un pH situé entre 4 et 8 et d'un composé cuivrique, soit un chauffage à un pH acide (inférieur à 4) en présence ou non d'un composé du cuivre.

C'est donc la conjonction des différentes caractéristiques du procédé (température, pH, composé cuivrique) qui permet l'élimination de tous les peroxydes d'acétone.

La différence de comportement des 2 peroxydes de l'acétone induit que l'on peut procéder :
- soit en une étape pendant laquelle le mélange réactionnel à traiter est amené au pH désiré, additionné du composé cuivrique et porté à la température choisie ;
- soit en 2 étapes successives consistant à commencer le chauffage du mélange réactionnel à traiter à un pH inférieur à 4, pendant quelques minutes à quelques heures, en présence ou non du composé du cuivre, puis à ajuster le pH à la valeur désirée de 4 à 8 après refroidissement du mélange réactionnel et ajout éventuel du composé du cuivre, s'il n'a pas été introduit précédemment, enfin à reprendre le chauffage à la même température que précédemment ou à une température plus élevée.

Le mélange réactionnel traité par le procédé selon l'invention, peut ensuite être soumis aux différentes phases habituelles de séparation des produits qu'il contient : distillation, recristallisation par exemple.

### EXEMPLE 1: Préparation d'un mélange réactionnel contenant des peroxydes d'acétone

Dans le cadre de la synthèse du phénol par oxydation du cumène, on obtient un oxydat brut contenant, après concentration, essentiellement de l'hydroperoxyde de cumyle (HPOC) et du phényl-2 propanol-2.

Cet oxydat brut est soumis à une opération de scission acide de l'HPOC en phénol et acétone, après que l'on y ait ajouté la quantité de solution aqueuse à 70 % en poids de H₂O₂, nécessaire pour oxyder le phényl-2 propanol-2 en HPOC.

La scission de l'HPOC est effectuée par injection simultanée de l'oxydat et d'acide sulfurique concentré, dans un réacteur en acier inoxydable comportant un trop-plein.

Le réacteur dans lequel s'effectue cette opération de scission acide est muni d'une double enveloppe reliée à un bain thermostaté, afin de permettre l'élimination des calories dégagées par la réaction.

### Conditions de la scission :

- rapport molaire H₂O₂/phényl-2 propanol-2 = 1
- rapport pondéral H₂SO₄/oxydat brut = 1,25 %
- température maintenue à 57°C
- temps moyen de séjour dans le réacteur = 7,4 minutes.

L'oxydat final est dosé par chromatographie en phase gazeuse.

En dehors des composés majoritaires (phénol et acétone), on trouve :
- peroxyde d'acétone dimère : 0,4200 % en poids (limite de détection : 0,0200 %)
- peroxyde d'acétone trimère : 0,0400 % en poids (limite de détection : environ 0,0100 %)

### EXEMPLE 2 : Destruction des peroxydes d'acétone dans l'oxydat préparé dans l'exemple 1

Dans un ballon en verre de 30 cm3, résistant à la pression, muni d'un réfrigérant ascendant, on charge 20 g de l'oxydat final traité dans l"exemple 1.

On chauffe à 57°C pendant 30 minutes, puis on refroidit l'essai et on neutralise le mélange à pH 5,6 (tel que défini dans le présent texte) à l'aide d'une solution 7,7 N de soude.

On ajoute alors 0,014 g d'acétate cuivrique (0,1134 mmol).

On chauffe sous agitation à 110°C pendant 20 minutes, sous pression autogène.

Par dosage en chromatographie en phase gazeuse (CPG) on constate la disparition totale du peroxyde d'acétone dimère et du peroxyde d'acétone trimère (dans les limites de détection du dosage indiquées précédemment).

### EXEMPLE 3 : Destruction des peroxydes d'acétone dans l'oxydat préparé dans l'exemple 1

On répète l'exemple 2, en modifiant la phase de chauffage après l'ajustement du pH à 5,7 et l'addition de l'acétate cuivrique : on chauffe sous agitation et sous pression autogène pendant 1 heure 45 min à 73°C.

Par dosage en CPG, on constate la disparition totale (dans les limites de détection indiquées précédemment) du peroxyde d'acétone dimère et du peroxyde d'acétone trimère.

### EXEMPLE 4: Destruction des peroxydes d'acétone dans l'oxydat préparé dans l'exemple 1

Dans un ballon en verre de 30 cm3, résistant à la pression, on charge 20 g de l'oxydat final traité dans l"exemple 1.

On ajoute de la soude concentrée (7,7 N) jusqu'à pH 5,8 (mesuré dans les conditions définies dans le présent texte).

On filtre le sulfate de sodium formé et on ajoute 0,01 g (0,1134 mmol) d'acétate de cuivre.

On chauffe sous agitation pendant 1 heure 30 minutes à 110°C, sous pression autogène.

Par dosage en CPG, on constate la disparition totale (dans les limites de détection indiquées précédemment) du peroxyde d'acétone dimère et du peroxyde d'acétone trimère.

### Essai comparatif A

On répète l'exemple 2, mais on charge l'acétate de cuivre dans l'oxydat au début de l'essai et on ne modifie pas la valeur du pH (1,6).

On chauffe sous agitation et sous pression autogène pendant 1 heure à 57°C.

Par dosage en CPG on trouve dans le mélange réactionnel final :
- 0,3990 % de peroxyde d'acétone dimère (environ 95 % de la quantité présente avant le traitement)
- le peroxyde d'acétone trimère a disparu (dans les limites de détection indquées précédemment).

### EXEMPLE 5 : Préparation d'un mélange réactionnel contenant des peroxydes d'acétone

Dans un ballon tricol en verre de 500 cm3, muni d'une agitation centrale et d'une ampoule de coulée, on charge :
- 100 g (1,064 mol) de phénol
- 78 g d'acétone
- 5 g d'une silicalite de titane (préparée selon le brevet français FR 2 471 950) ayant un rapport molaire TiO₂/SiO₂ d'environ 3 %.

On agite et on chauffe à 80°C, puis on introduit 20 cm3 d'une solution aqueuse à 36 % en poids/volume de H₂O₂ (0,2 mol).

Lorsque H₂o₂ a été consommé, on sépare le catalyseur par filtration et on dose dans le mélange réactionnel 5A, par chromatographie en phase gazeuse (CPG), à côté des composés majoritaires (phénol non transformé, acétone, diphénols) :
- 0,0065 % en poids de peroxyde d'acétone dimère
- 0,0200 % en poids de peroxyde d'acétone trimère.

Le catalyseur utilisé précédemment et récupéré est engagé dans 2 nouvelles opérations successives d'hydroxylation du phénol.

On dose en CPG dans ces 2 essais 5B et 5C les peroxydes d'acétone.

### Essai 5B :

- peroxyde d'acétone dimère : 0,0170 % en poids
- peroxyde d'acétone trimère : 0,0250 % en poids

### Essai 5C :

- peroxyde d'acétone dimère : 0,0340 % en poids
- peroxyde d'acétone trimère : 0,0650 % en poids

### EXEMPLE 6

On charge 18,0 g de mélange réactionnel 5C préparé dans l'exemple 5, dans l'appareillage décrit dans l'exemple 2.

On amène le pH (mesuré dans les conditions définies dans le présente texte) à une valeur de 5,2.

On introduit alors 0,028 g d'acétate de cuivre et on chauffe à 110°C pendant 1 heure sous pression autogène.

Par dosage en CPG, on constate la disparition totale (dans les limites de détection indiquées précédemment) du peroxyde d'acétone dimère et du peroxyde d'acétone trimère.

## Revendications

1. Procédé de destruction des peroxydes d'acétone présents dans un mélange réactionnel, issu notamment de la synthèse du phénol, de diphénols ou de dérivés du phénol, caractérisé en ce que :
- l'on amène le pH dudit mélange réactionnel à une valeur de 4 à 8 ;
- l'on ajoute audit mélange un composé du cuivre ;
- et l'on maintient ledit mélange à une température de 50°C à 150°C.

2. Procédé selon la revendication 1, caractérisé en ce que le composé du cuivre est choisi parmi les composés cuivreux ou cuivriques, au moins partiellement solubles dans le mélange réactionnel à traiter.

3. Procédé selon l'une des revendications 1 ou 2, caractérisé en ce que le composé du cuivre est choisi parmi les carboxylates cuivriques, les halogénures cuivriques, le sulfate de cuivre et le nitrate de cuivre.

4. Procédé selon l'une des revendications 1 à 3, caractérisé en ce que le composé du cuivre utilisé est l'acétate cuivrique.

5. Procédé selon l'une des revendications 1 à 4, caractérisé en ce que l'on met en oeuvre de 0,05 mole à 5 moles de composés du cuivre pour 1 mole de peroxyde d'acétone et de préférence de 0,1 mole à 2 moles de composé du cuivre pour 1 mole de peroxyde d'acétone.

6. Procédé selon l'une des revendications 1 à 5, caractérisé en ce que le pH du mélange réactionnel à traiter est ajusté entre 5 et 7.

7. Procédé selon l'une des revendications 1 à 6, caractérisé en ce que l'on opère entre 60°C et 130°C.

8. Procédé selon l'une des revendications 1 à 7, caractérisé en ce que le mélange réactionnel à traiter est amené à un pH compris entre 4 et 8, additionné de composé cuivrique et porté à une température de 50°C à 150°C.

9. Procédé selon l'une des revendications 1 à 7, caractérisé en ce que l'on chauffe le mélange réactionnel à traiter pendant quelques minutes à quelques heures à un pH inférieur à 4, en présence ou non du composé du cuivre, que l'on ajuste le pH à la valeur désirée de 4 à 8 après refroidissement et que l'on ajoute le composé du cuivre s'il n'a pas été introduit précédemment, enfin que l'on reprenne le chauffage à la même température que précédemment ou à une température plus élevée comprise entre 50°C et 150°C.

10. Procédé selon l'une des revendications 1 à 9, caractérisé en ce que le mélange réactionnel à traiter provient de l'oxydation du cumène pour la synthèse du phénol.

11. Procédé selon l'une des revendications 1 à 9, caractérisé en ce que le mélange réactionnel à traiter provient de l'oxydation du diisopropyl-1,4 benzène pour la synthèse de d'hydroquinone.

12. Procédé selon d'une des revendications 1 à 9, caractérisé en ce que le mélange réactionnel à traiter provient de l'hydroxylation du phénol, de phénols substitués ou d'éthers de phénol pour la synthèse de diphénols, de diphénols substitués ou de phénols substitués.

## Claims

1. Process for the destruction of the acetone peroxides present in a reaction mixture, resulting in particular from the synthesis of phenol, diphenols or phenol derivatives, characterised in that:
- the pH of the said reaction mixture is brought to a value of 4 to 8,
- a copper compound is added to the said mixture
- and the said mixture is maintained at a temperature of 50°C to 150°C.

2. Process according to claim 1, characterised in that the copper compound is chosen from the cuprous or cupric compounds which are at least partially soluble in the reaction mixture to be treated.

3. Process according to one of claims 1 or 2, characterised in that the copper compound is chosen from the cupric carboxylates, the cupric halides, copper sulphate and copper nitrate.

4. Process according to one of claims 1 to 3, characterised in that the copper compound used is cupric acetate.

5. Process according to one of claims 1 to 4, characterised in that from 0.05 mole to 5 moles of copper compounds are used for 1 mole of acetone peroxide, and preferably from 0.1 mole to 2 moles of copper compound for 1 mole of acetone peroxide.

6. Process according to one of claims 1 to 5, characterised in that the pH of the reaction mixture to be treated is adjusted to between 5 and 7.

7. Process according to one of claims 1 to 6, characterised in that it is carried out at between 60°C and 130°C.

8. Process according to one of claims 1 to 7, characterised in that the reaction mixture to be treated is brought to a pH of between 4 and 8, a cupric compound is added and the mixture is brought to a temperature of 50°C to 150°C.

9. Process according to one of claims 1 to 7, characterised in that the reaction mixture to be treated is heated for a few minutes to a few hours at a pH of less than 4, in the presence or absence of the copper compound, that the pH is adjusted to the required value of 4 to 8 after cooling, and that optionally the copper compound is added if it has not already been introduced, lastly that heating is resumed to the same temperature as above or to a higher temperature of between 50°C and 150°C.

10. Process according to one of claims 1 to 9, characterised in that the reaction mixture to be treated comes from the oxidation of cumene for the synthesis of phenol.

11. Process according to one of claims 1 to 9, characterised in that the reaction mixture to be treated comes from the oxidation of 1,4-diisopropylbenzene for the synthesis of hydroquinone.

12. Process according to one of claims 1 to 9, characterised in that the reaction mixture to be treated comes from the hydroxylation of phenol, of substituted phenols or of phenol ethers for the synthesis of diphenols, of substituted diphenols or of substituted phenols.

## Patentansprüche

1. Verfahren zur Vernichtung von Acetonperoxiden in einem Reaktionsgemisch, das insbesondere aus der Synthese von Phenol, von Dihydroxybenzolen oder von Phenolderivaten hervorgeht, dadurch gekennzeichnet, daß:
- der pH des genannten Reaktionsgemischs auf einen Wert von 4 bis 8 eingestellt wird;
- zu dem genannten Gemisch eine Kupferverbindung gegeben wird;
- und das genannte Gemisch bei einer Temperatur von 50°C bis 150°C gehalten wird.

2. Verfahren nach Anspruch 1, dadurch gekennzeichnet, daß als Kupferverbindung eine Kupfer(I)- oder eine Kupfer(II)-Verbindung gewählt wird, die mindestens partiell in dem aufzubereitenden Reaktionsgemisch löslich ist.

3. Verfahren nach einem der Ansprüche 1 und 2, dadurch gekennzeichnet, daß die Kupferverbindung ein Kupfer(II)-carboxylat, ein Kupfer(II)-halogenid, Kupfer(II)-sulfat oder Kupfer(II)nitrat ist.

4. Verfahren nach einem der Ansprüche 1 bis 3, dadurch gekennzeichnet, daß als Kupferverbindung Kupfer(II)-acetat verwendet wird.

5. Verfahren nach einem der Ansprüche 1 bis 4, dadurch gekennzeichnet, daß 0,05 bis 5 Mole der Kupferverbindung pro Mol Acetonperoxyd, vorzugsweise 0,1 bis 2 Mole pro Mol Acetonperoxyd eingesetzt werden.

6. Verfahren nach einem der Ansprüche 1 bis 5, dadurch gekennzeichnet, daß der pH des aufzubereitenden Reaktionsgemischs auf einen Wert zwischen 5 und 7 eingestellt wird.

7. Verfahren nach einem der Ansprüche 1 bis 6, dadurch gekennzeichnet, daß die Arbeitstemperatur zwischen 60°C und 130°C beträgt.

8. Verfahren nach einem der Ansprüche 1 bis 7, dadurch gekennzeichnet, daß der pH des aufzubereitenden Reaktionsgemischs auf einen Wert von 4 bis 8 eingestellt wird, sowie das genannte Gemisch mit einer Kupfer(II)-verbindung versetzt und auf eine Temperatur von 50°C bis 150°C gebracht wird.

9. Verfahren nach einem der Ansprüche 1 bis 7, dadurch gekennzeichnet, daß das aufzubereitende Reaktionsgemisch in An- oder Abwesenheit der Kupferverbindung für einige Minuten bis einige Stunden bei einem pH-Wert kleiner als 4 erwärmt wird, daß der pH nach dem Abkühlen auf einen Wert von 4 bis 8 eingestellt wird und - falls die Kupferverbindung nicht schon zuvor hinzugefügt wurde - die Kupferverbindung zugegeben wird, und daß das Gemisch schließlich auf die Anfangstemperatur oder auf eine höhere Temperatur von 50°C bis 150°C erhitzt wird.

10. Verfahren nach einem der Ansprüche 1 bis 9, dadurch gekennzeichnet, daß die aufzubereitende Reaktionsmischung aus der Oxidation von Kumol bei der Phenolsynthese hervorgeht.

11. Verfahren nach einem der Ansprüche 1 bis 9, dadurch gekennzeichnet, daß das aufzubereitende Reaktionsgemisch aus der Oxidation von 1,4-Diisopropylbenzol bei der Synthese von Hydrochinon hervorgeht.

12. Verfahren nach einem der Ansprüche 1 bis 9, dadurch gekennzeichnet, daß das aufzubereitende Reaktionsgemisch aus der Hydroxylierung von Phenol, von substituierten Phenolen oder von Phenolethem bei der Synthese von Dihydroxybenzolen, substituierten Dihydroxybenzolen oder substituierten Phenolen hervorgeht.
